# EUROPEAN PATENT SPECIFICATION

(11) **EP 0 692 027 B1**
(45) Date of publication and mention of the grant of the patent: **16.06.2004**
(21) Application number: 94912039.8
(22) Date of filing: 08.04.1994
(51) Int. Cl.: C12N 15/12, C12N 15/62

(54) **EXPRESSION OF BETA-2-MICROGLOBULIN IN PICHIA PASTORIS**
EXPRESSION VON BETA-2-MIKROGLOBULIN IN PICHIA PASTORIS
EXPRESSION DE BETA-2-MICROGLOBULINE DANS $i(PICHIA PASTORIS)

(30) Priority: 08.04.1993 GB 9307371
(43) Date of publication of application: 17.01.1996
(73) Proprietor: ISIS INNOVATION LIMITED, Oxford OX1 2JD (GB)
(72) Inventor: HUNTER, Michael George, British Biotech Phar. Ltd., Cowley, Oxford OX4 5LY (GB); WOODS, Nigel Robert, British Biotech Pharm. Ltd., Cowley, Oxford OX4 5LY (GB)
(74) Representative: Roques, Sarah Elizabeth
(86) International application number: PCT/GB1994/000754
(87) International publication number: WO 1994/024280

(56) References cited:
- EP-A- 0 495 208
- GENE. vol. 83 , 1989 , AMSTERDAM NL pages 117 - 124 K. PARKER AND D. WILEY 'Overexpression of native human beta-2-microglobulin in Escherichia coli and its purification'
- NATURE. vol. 347 , 25 October 1990 , LONDON GB pages 770 - 772 H. BROWNE TE AL 'A complex between the MHC class I homologue encoded by human cytomegalovirus and beta 2 microglobulin'
- C. HERSHBERGER ET AL (EDS) 'Genetics and molecular biology of industrial microorganisms; fourth ASM' 1989 , AMERICAN SOCIETY FOR MICROBIOLOGY , WASHINGTON J. CREGG ET AL 'Expression of foreign genes in Pichia pastoris' see page 343 - page 352

## Description

This invention concerns the efficient preparation of recombinant beta-2 microglobulin.

Immune responses have two major components, humoral and cell-mediated. The first comprises antibodies, produced by B-lymphocytes, which bind to specific antigens and which serve a number of important functions such as neutralisation of viruses, complement fixation and immune complex formation. Antibody production is dependent on helper T cells, which mediate the antigen specific recognition of peptide epitopes displayed by Class II Major Histocompatibility Complex (MHC) molecules on the surface of antigen presenting cells.

The second arm of the immune system involves effector T cells, in particular cytotoxic T lymphocytes (CTLs) which can recognise and kill infected or transformed cells. Such responses are particularly important in the defense against and immunity from viral infections, though they are also involved in host responses against certain tumours. CTL responses are also antigen specific, involving subsets of CD8 positive T cells. Immune recognition is mediated by the T cell receptor which recognises the peptide antigen displayed on Class I MHC molecules.

Class I MHC molecules are ubiquitously expressed and consist of two polypeptide chains. The first is an alpha chain of about 45kDa comprising 3 domains. Two of these domains bind to peptides derived from processing endogenously synthesised proteins, such as viral components, and present them to the T cell receptor. These two domains are linked to a single membrane spanning anchor region by a third, immunoglobulin-like domain. The second component is beta-2 microglobulin (B2M), a 100 amino acid protein which can exist free in the serum as well as a part of Class I MHC. The two chains normally associate in the ER, along with peptides produced by the degradation of endogenous proteins to form a ternary complex which is displayed on the cell surface. Although binary complexes lacking peptide can be formed, these are unstable and are normally recycled or degraded (Ljungren et al. Nature 346, 476- (1990).

Crystallographic data reveals that the antigenic peptide binds to a groove between the first two domains of the alpha subunit, the base of which is formed from strands of beta sheet, and the walls from two alpha helices (Bjorkman et al. Nature 329, 506-512 (1987)). The nature of the side chains in this region of the alpha chain are critical in determining the peptide selectivity of the molecule, and hence the ability of different Class I MHC alleles to respond to particular epitopes (Bjorkman et al. Nature 329, 512-518 (1987)). Although it is the alpha subunit that binds the peptide, B2M plays an essential role in allowing binding, presumably by stabilising the ternary complex (Vitiello et al. Science 250, 1423-1426 (1990); Rock et al. PNAS 88, 310-304 (1991)). While some free alpha subunit can reach the cell surface in the absence of B2M, and can bind and present peptide, it is considerably less efficient than in the presence of B2M (Bix and Raulet. Exp. Med. 176, 829-834 (1992).

Although the normal route of antigen presentation by Class I MHC involves the degradation of endogenously synthesised proteins, it is possible to achieve antigen presentation by addition of high concentrations of peptide to antigen presenting cells (APC) *in vitro.* The use of peptides as immunogens has a number of potential advantages, the most significant of which are that it avoids the use of inactivated or attenuated viral particles, and that small peptides can be synthesised chemically, avoiding the requirement for biological production routes. The most efficient presentation is obtained with peptides of from 9-11 residues in length, which corresponds to the length of the groove if they are in an extended conformation.

However, there are major problems associated with this method of eliciting a CTL response. Although such a use of peptides is adequate for demonstrating immune responses such as T cell-mediated killing *in vitro,* it is not an efficient process, and does not offer a practical route to *in vivo* immunisation. CTL responses may be induced by immunisation with certain small peptide fragments but such peptides may elicit poor antibody responses. Further, in an outbred population, individuals of different MHC class I and class II haplotypes will respond to different peptide epitopes; many peptides may be required to prime all the possible haplotypes.

Most of the available evidence also indicates that immunisation with whole proteins does not give rise to a CTL response. Such CTL responses that have been induced require the presence of powerful adjuvants or live vector which have not been licensed for use in humans. For example, recombinant HIV gp160 presented in ISCOMs, but not in FCA, FIA or buffered saline, induced HIV-1 envelope-specific CTL activity in BALB/c mice. It is generally felt that approval for the use of ISCOMs in humans is unlikely to be forthcoming. Recombinant vaccinia viruses expressing the V3 region of the HIV envelope gp160 also induce specific CTL responses to the V3 region, but immunisation with vaccinia has raised concerns both about safety for immunocompromised patients, and about efficacy. A simpler and more efficient way to induce CTL responses is therefore an important goal, and has been sought actively for a number of years though with only limited success.

It has been shown that the exchange of B2M chains in MHC class I will occur in the presence of free B2M (Hayfil and Strominger, PNAS USA 76(11) 5834-5838 (1979)), and that the binding of exogenous peptides to these molecules occurs upon association and reassociation of B2M light chains (Rock et al., PNAS USA 87 7517-7521 (1990); Kozlowski et al. Nature 349, 74-77 (1991)). The presentation of exogenously added peptide can therefore be made more efficient by the addition of B2M. The use of exogenous B2M in this way to enhance immune responses against peptides *in vivo* is described in WO-A-91/16924.

It would therefore be desirable if B2M were available in quantity, to capitalise on its potential in enhancing the immunogenicity of peptide containing vaccine compositions. In principle, B2M for use in the enhancement of immune responses could be purified from natural sources such as serum or urine. However, this difficult and expensive. Also, blood products must be screened for a number of contaminants before being acceptable for use in vaccines. A source of recombinant B2M would solve these problems, but despite the DNA sequence of B2M being known, to date it has not been possible to produce recombinant B2M efficiently. Extremely low yields are produced by expression in mammalian cells, and the protein is incorrectly folded following expression in *E. coli.* Nor are yeasts a universally host for the purpose. The present inventors have found that standard techniques for obtaining expression of B2M in perhaps the most widely used yeast for such purposes, namely *S. cerevisiae,* resulted in very poor yields.

The *Pichia pastoris* expression system is well known, and has particular advantages in its ease of scalability for large scale production. High level expression has been obtained for a number of proteins in that host, but equally, some have proved difficult to produce. There is no obvious correlation between the properties of a particular polypeptide and its ability to be highly expressed in the *Pichia* system.

In contrast with their experience of low level expression of B2M in *S. cerevisiae,* the present inventors have found that surprisingly high yields can be obtained in the *Pichia* system.

Therefore, according to the invention there is provided a method method of producing recombinant beta-2-microglobulin (B2M) by cultivating a methylotropic yeast harbouring an expression vector comprising DNA encoding B2M, wherein the B2M is expressed as a fusion of B2M with a Pho1 secretary leader, and the resultant fusion is cleaved by endogenous endoproteinase(s) present in the growth medium to release B2M into the medium and recovering expressed B2M.

Methylotropic yeast strains include *Pichia,* in particular *P. pastoris, Hansenula, Candida* and *Torulopsis.* Use *of Pichia pastoris* is presently preferred.

Recombinant DNA encoding B2M may be incorporated in a yeast expression vector for expression in methylotropic yeast in accordance with the invention. Such vectors will usually contain a promoter. PGK is a preferred promoter, but any other suitable promoter may be used if necessary or desirable. Examples include GAPD, GAL1-10, PHOS, ADH1, CYC1, Ty delta sequence, PYK and hybrid promoters made from components from more than one promoter (such as those listed).

To obtain secretion of the B2M from the yeast cells after expression, the expression vector includes a secretion leader sequence fused to the B2M sequence. After secretion, the leader sequence is automatically cleaved from the B2M protein by enzyme(s) produced naturally during the cultivation of the transformed yeast cells. Such secretion techniques are well known. Secretion leaders known to the art include the alpha factor, Phol, HSA and Suc2. However, the present inventors have found that when the yeast alpha factor secretion signal is used, cleavage is not 100% accurate. the final yield of B2M was contaminated with a fusion of B2M with part of the alpha factor sequence, indicating incomplete removal of the alpha factor leader. Although B2M may be separated from the contaminant by standard purification methods, for example those based on differing molecular weights, it would be desirable to avoid the difficulty if possible.

The inventors have found that the Pho1 leader is correctly cleaved, and it is therefore the presently preferred secretion factor for use in accordance with the invention.

Yields of more than 0.5g/L, for example about 1g/L or more are feasable with the present invention. The B2M produced is correctly folded, and essentially indistinguishable from native B2M.

The invention is illustrated by the following Examples:

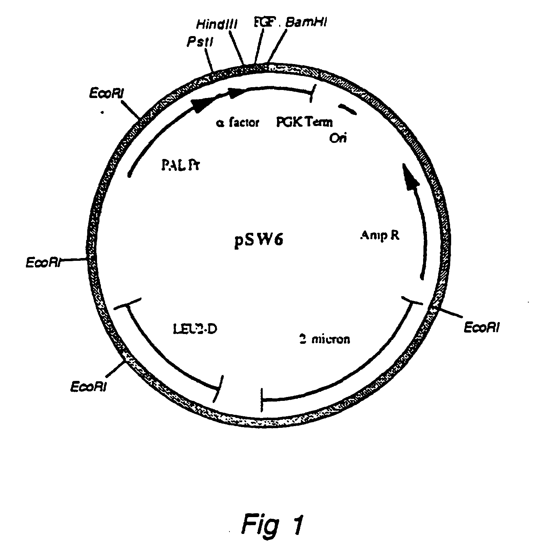

**Figure 1** shows the expression vector used for the attempted expression of B2M in *S.cerevisiae.*
**Figure 2** shows the *Pichia* expression vector pHILD1 used as the starting point for the construction of the expression vector for high-level expression of B2M in *P.pastoris,* using the alpha factor leader sequence.
**Figure 3** shows the intermediate vector pLHD4 used in the construction of the *P*. *pastoris* alpha factor/B2M expression vector.
**Figure 4** shows the *P. pastoris* alpha factor/B2M expression vector pLH15.
**Figure 5** shows the *Pichia* expression vector pHILS1 used as the starting point for the construction of the expression vector for high-level expression of B2M in *P.pastoris,* using the Pho1 leader sequence, and the manipulations leading to the final P. pastoris Pho1/B2M expression vector pLH46.
**Figure 6** shows the the sequence of the Pho1/B2M fusion included in pLH46 (Figure 5), where the underlined sequence represents the cloning site, the italics represent the Pho1 signal sequence, and the bold letters are identical to the sequence of the B2M gene.

### Example 1 - Isolation of the B2M gene and preparation for cloning

RNA was purified from human blood cells using the isothiocyanate guanidinium method (RNAzol B^{TM}, Biogenesys). This RNA was used as the template for synthesis of first strand cDNA with the use of oligo dT primers using the method of Maniatis (in Molecular Cloning, 1989).

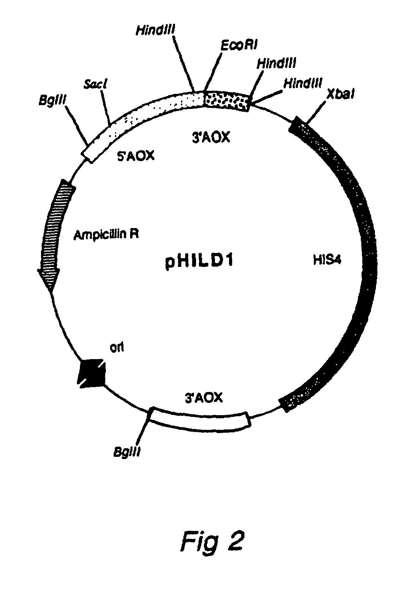

The cDNA was then amplified in PCR reactions using primer (5'-GACAAGCTTGGATAAAAGAATCCAGCGTACTCCAAAG-3') SEO ID 1 to add a *Hind*III restriction site and an adaptor sequence encoding the last 5 amino acids of the *S.cerevisiae* alpha mating factor at the 5' end of the gene, and (5'-CATAGGATCCTTATTACATGTCTCGATCCCACTT-3') SEQ ID 2 that added a termination codon and a *Bam*H1 site at the 3' end of the gene. PCR products were generated in 30 cycles of denaturation at 97°C for 1 min, annealing at 60°C for 1 min and elongation 72°C for 30 seconds. The reactions were carried out in 1 mM MgCl2, 50mM KCI, 10mM Tris pH 8.3 using 50ng of primer in a volume of 50µl. The sequence of the gene is shown in Figure 2. After PCR amplification the DNA was digested with *Hind*III and *Bam*H1 and ligated into *Hind*III*-Bam*HI calf intestinal phosphatase-treated M13mp19. M13 clones were then sequenced to identify isolates that had no difference to the published sequence using universal primer (5'-GTTTTCCCAGTCACGAC-3') SEQ ID 3. DNA was then purified from one of these clones and subcloned into the *S.cerevisiae* expression vector pSW6 (see Example 2).

### Example 2 - Cloning the beta-2-microglobulin gene into the S.cerevisiae expression vector pSW6

An expression vector was designed to enable secretion of B2M to the extracellular medium after expression in *S. cerevisiae.* Secretion aids purification and rapid analysis of B2M. The secretion signals from the yeast mating type factor alpha were used to direct export of the B2M.

The yeast expression vector pSW6 (Figure 2) is based on the 2 micron circle from *S. cerevisiae.* (pSW6 is deposited in *S. cerevisiae* strain BJ2168 at the National Collection of Industrial and Marine Bacteria Limited, 23 St. Machar Drive, Aberdeen AB2 1RY, United Kingdom under Accession No. NCIMB 40326). pSW6 is a shuttle vector capable of replication in both *E. coli* and *S. cerevisiae* and contains an origin of DNA replication for both organisms, the *leu*2 gene (a selectable marker for plasmid maintenance in the yeast host) and the ampicillin resistance locus for selection of plasmid maintenance in *E. coli.* (The DNA sequence for the vector is dsiclosed in WO-A-09125). The ability to passage this vector through *E. coli* greatly facilitates its genetic manipulation and ease of purification. pSW6 contains an alpha-factor pre-pro-peptide gene fused in-frame to a gene encoding human epidermal growth factor (EGF). The expression of this fusion is under the control of an efficient galactose regulated promoter which contains hybrid DNA sequences from the *S. cerevisiae* GAL 1-10 and phosophoglycerate kinase (PGK) promoters. Transcription of the EGF gene is terminated in this vector by the natural yeast PGK terminator. The EGF gene in pSW6 can be removed by digestion with restriction endonucleases *Hin*dIII and *Bam*HI. This removes DNA encoding both EGF and 5 amino acids from the C-terminus of the alpha-factor pro-peptide. Genes to be inserted into the pSW6 expression vector must therefore have the general composition: *Hind*III site-alpha factor adaptor-gene-*Ham*HI site. The B2M gene prepared in Example 1 has this general composition.

After digestion with *Hin*dIII and *Bam*HI endonucleases, the pSW6 vector contains the alpha factor gene minus the codons for the last five amino acid residues. The β2-microglobulin gene prepared as in Example 1 was then cloned into the pSW6 vector. The resulting vector was then transformed into the host strain BJ2168 using the method of Ito *et al* J. Bacteriol. (1983) **153** 163-168.

### Example 3 - Expression of B2M in S.carevisiae

Single colonies of the transformed strain from Example 2 were used to inoculate 5ml of sc/glc/aa (-leu) medium and the cultures grown overnight at 30°C on an orbital shaker. All of this starter culture was then used to inoculate a 500mL flask containing 100mL of the same medium. This culture was allowed to grow for 24 hours. Expression of the B2M gene was induced by resuspending the cells in fresh medium containing additional galactose (0.2%). The culture was allowed to grow for 72 hours before material was recovered from the culture supernatant. Cells were removed by centrifugation at 3000rpm for 5min.

Analysis of material by PAGE suggested expression levels to be in the order of only 1 mg/L. The protein seemed to form aggregates as other bands were detected in the supernatant corresponding to dimers and higher order

### Example 4 - Construction of alpha factor/B2M Pichia pastoris Expression Vector

The expression vectors used for this work were derived from the Phillips Petroleum expression vector pHILD1.

*Pichia* expression vector pHILD1 (Figure 2) is a shuttle vector capable of propagation in both *E. coli* (for ease of genetic manipulation) and in the methylotrophic yeast *Pichia pastoris.-* pHILD1 can be obtained under licence from the Phillips Petroleum Company, Bartlesville Oklahoma, USA. The vector comprises sequences derived from the *E.coli* vector pBR322 and sequences derived from the genome of *Pichia pastoris.* The essential features are the 5' region of the *Pichia* AOX gene including the regulatable AOX promoter for high level transcription, the 3' region from the AOX gene containing the alcohol oxidase transcriptional terminator sequence, a further region from the 3' part of the ~AOX gene is included which together with the 5' AOX region is required for site directed integration of the expression cassette into the host genome. The *P. pastoris* histidinol dehydrogenase gene HIS4 is carried and used to complement the defective HIS 4 gene in *Pichia* host strains. The ampicillin resistance gene is carried to enable selection in the *E.coli* hosts used during genetic manipulation.

The pHILD1 vector was manipulated to allow expression of the B2M gene under the control of the alpha factor secretion signal. pHILD1 does not carry any sequences encoding secretion signals to allow export of heterologous proteins. To include such a signal, the vector was manipulated by the addition of sequences from the *S.cerevisiae* alpha factor leader. The vector was further engineered to provide a more optimal promoter context and to remove undesirable *Hind* III restriction sites which may interfere with the cloning of the B2M gene; a *Bam* HI site was then introduced 3' to the remaining *Hind* III to allow cloning of the B2M gene on a *Hin*dIII - *Bam*HI restriction site and to include a kanamycin resistance cassette enabling the selection of multicopy integrants in transformed *Pichia* host strains. The stages of the manipulations are shown below.

### 1) Inclusion of alpha factor secretion signals

The alpha factor sequences were cloned into the pHILD1 vector from the *S. cerevisiae* expression vector pSW6 (Figure 2) (see example 3 for details). The alpha factor sequences were isolated from pSW6 on ca 430 bp *Bgl* II*-Bam* HI DNA fragment, this fragment contains the alpha factor sequences fused to a human epidermal growth factor synthetic gene (EGF). The overhanging ends of this DNA fragment were first filled in using klenow fragment of *E.coli* DNA polymerase I together with the required deoxynucleoside triphosphates according to standard methodology. The flush ended fragment was then cloned into the pHILD1 vector that had been treated with *Eco* RI and then blunt ended as above. The integrity of the resultant plasmid pLH001 was checked by a combination of restiction digestion and DNA sequence analysis. The primer use for sequence analysis was (5'-GCATTCTGACATCCTCT-3'), SEQ ID 4. The sequence of the alpha factor fusion was confirmed.

### 2) Mutagenesis to optimise vector for B2M expression

To remove unwanted *Hind* III restriction sites, optimise the promoter region and introduce a *Bam* H1 site from the pLHOO1 vector, relevant fragments were cloned into bacteriophage M13 for site directed mutagenesis. The fragments cloned, the primers used for mutagenesis, and the primers used for sequencing are detailed below. Furthermore, a kanamycin resistance cassette was modified for introduction into the final expression vector to allow selection for muticopy integrants when the vector is introduced into *Pichia* host strains.

A 1220 bp *Sac*I-*Sac*I fragment was isolated from pLH001 and cloned into M13 mp19. This M 13 construct was then used for mutagenesis in which a *Hind* III site was removed using oligonucleotide primer (5'-CGTTAAAATCAACAACTTGTCAATTG-GAACC-3'), SEQ ID 5, the mutants were identified by sequence analysis with sequencing primer (5'-GGAAATCTCACAGATCT-3'), SEQ ID 6. This fragment was further modified by deletion mutagenesis to optimise the 5' untranslated leader region preceeding the AOX promoter, which is now identical to that found in the natural 5' untranslated leader of the AOX1 gene on the *Pichia* genome. Having the correct context around the 5' untranslated leader is preferred for maximal expression. The mutagenesis primer used for this step was (5'GAAGGAAATCTCATCGTTTCGAATA-3'), SEQ ID 7. The mutant was identified by sequence analysis with sequencing primer (5'-GCTAATGCGG-AGGATGC-3'), SEQ ID 8.

Two further *Hind* III sites was removed from from the 770bp Sac I-*Xba* I fragment of pLH001 by mutagenesis. The *Sac*I*-Xba*I fragment of pLH001 was first cloned into M13 mp18 and one of the *Hind* III sites was removed using the primer (5'-CCGGCATTACAACTTATCGATAAGCTTGCAC-3'), SEQ ID 9. The identity of this mutant was confirmed by sequence analysis using the sequencing primer (5'-GCGCATTGTTAGATTTC-3'). SEQ ID 10. A second *Hin*dIII site was removed from this newly mutagenised fragment using mutagenesis primer (5'-CTTATCGATCAACTTGCACAAACG-3'), SEQ ID 11. The correct mutant was identified by sequence analysis using sequence primer SEQ ID 9 (see above).

Before reassembly, a *Bam* HI site was introduced into the *Hind* III deleted *Sac I-Xba* I fragment to allow subsequent cloning of the B2M gene of example 1 on a *Hind* III-*Bam* HI fragment. The mutagenesis primer used to introduce the *Bam* H1 site was (5'-GTCATGTCTAAGGCGGATCCTTATTAAC-3'), SEQ ID 12. The identity of the mutant was identified using sequencing primer (5'-GCATTCTGACATCCTCT-3') SEQ ID 13.

### 3) Modification of the Kanamycin resistance cassette

A kanamycin resistance cassette was purchased from Pharmacia Biosystems Limited, Davy Avenue. Knowlhill, Milton Keynes, MK5 8PH. Great Britain. This cassette is supplied as an EcoRI fragment by Pharmacia and this was cloned into M13 mp19 as an *Eco*R1 fragment. The internal *Hind* III was deleted using mutagenesis primer (5'- GAGAATGGCAACAACTTATGCATT-3'), SEQ ID 14. The mutation was confirmed using sequencing primer (5'-CCAACATCAATACAACC-3'), SEQ ID 15.

### 4) Reassembly of expression vector

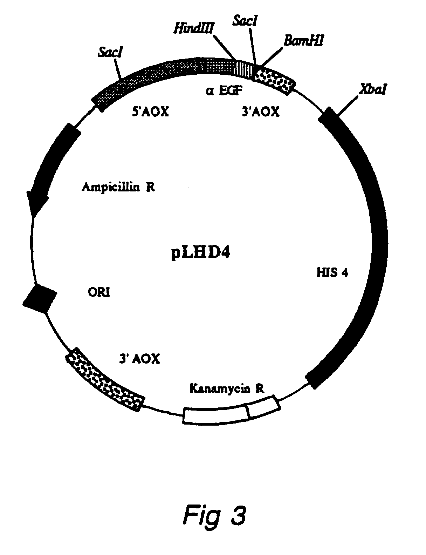

The vector was reconstructed in a stepwise manner using the Philips petroleum vector pHILD1 (Figure 3) as a backbone for the cloning.

To rebuild the expression vector including the mutagenised fragments, the modified ca 770 bp *Sac*l-*X*baI fragment was first ligated into *Sac*l -*Xba*l treated pHILD1 vector. The integrity of the recombinant construct was then confirmed by restriction analysis and DNA sequence analysis using the oligonucleotide sequencing primer (5'- GCGCATTGTTAGATTTC - 3'), SEQ ID 16, the construct was called intermediary vector 1. The modified *Sac* I-*Sac* I fragment was next cloned into intermediary vector 1 which had been treated with *Sac* I and calf intestinal phosphatase. The resultant construct named intermediary vector 2, was again confirmed by restriction analysis and DNA sequence analysis with oligonucleotide primers (5'-GGAAATCTCATAGATCT-3'), SEQ ID 17 to read through the deleted *Hind* III site and (5'-GCTAATGCGGAGGATGC-3'), SEQ ID 18 to read through the optimised 5' untranslated leader region. Intermediary vector 2 is a homologue of pHILD1 which lacks the unwanted *Hind* III sites, has an optimised 5' untranslated region, contains sequences encoding the *S. cerevisiae* alpha factor secretion signals followed by the remaining *Hind* III site and which has a *Bam* HI site 3' to the *Hind*III site to allow cloning of the B2M gene.

A 1200bp *Hinc*11 fragment containing the mutagenised kanamycin cassette was removed from the M13 mp19 mutagenesis vector (used to remove the *Hind* III site from the kanamycin resistance gene) and cloned into the unique *Nae* 1 site of the intermediary vector 2. The vector was renamed pLHD4. The integrity of pLHD4 was confirmed by restriction analysis. A map of pLHD4 is shown in Figure 3. pLHD4 contains the human EGF gene fused to the *S. cerevisiae* secretion signal.

### 5) Construction of the B2M Pichia expression vector

The final expression vector for B2 microglobulin expression was constructed by cloning the *Hind* III *- Bam* HI fragment of example 2 into pLHD4. (This 320bp*Hind* III-*Bam* HI fragment contains the B2M gene fused to the 3' end of a sequence encoding the 5 amino acids of the yeast alpha factor which precede the KEX2 cleavage site required for liberation of the mature peptide following secretion from the *Pichia* host).

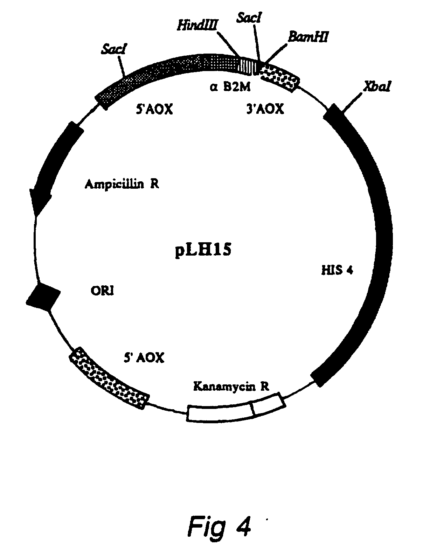

The *Hind* III-*Bam* H1 fragment was obtained by restriction digestion of the *S. cerevisiae* expression vector pSW6 82M. This fragment was purified on a 1.5% low melting temperature agarose gel then ligated to *Hind* III-*Bam* H1, calf intestinal phosphatase treated pLHD4. The resultant recombinant was called pLH15. The vector is shown on Figure 4. (At this time the Kanamycin resistance gene was defective, due to a then unknown deletion of bases, which occured during mutagenesis to delete the internal *Hindl* III site. A ca 2070 bp *Sac* I- *Xba* I fragment was removed from the resultant construct and cloned into the expression vector pHILD4, so conferring Kanamycin resistance) The integrity of the construct was confirmed by restriction analysis and sequencing analysis using the sequencing primer (5'-GCATTCTGACATCCTCT -3' ), SEQ ID 19.

### Example 5 - Construction of Pho1/B2M Pichia pastoris Expression Vector

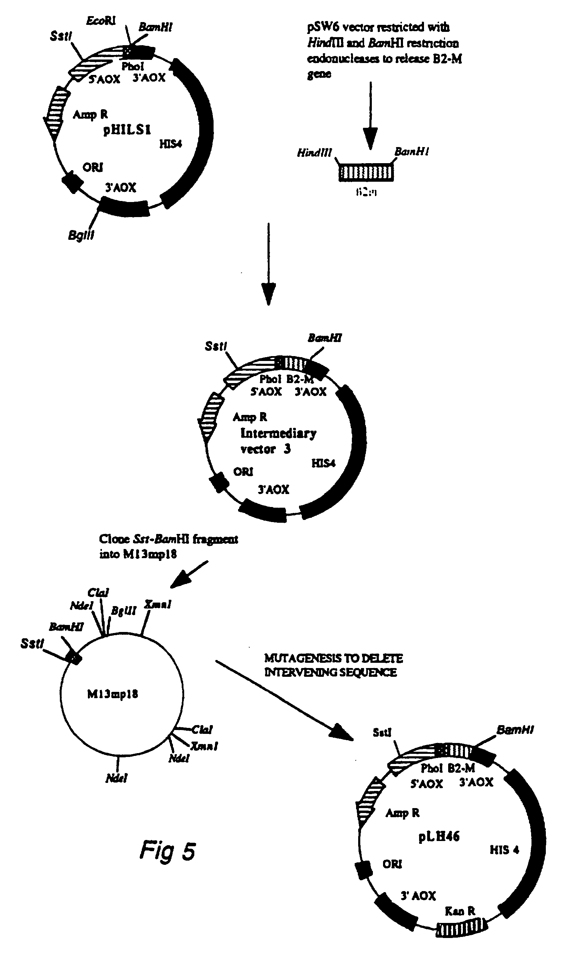

The expression vectors used for this work were derived from the Phillips Petroleum expression vector pHILS1 (Figure 5)

The starting expression vector for manipulation was the *Pichia pastoris* shuttle vector pHILSI (obtainable under licence from Phillips Petroleum) capable of propagation in both *E. coli* (for ease of genetic manipulation) and in the methylotropic yeast *Pichia pastoris.* It contains the Pho1 leader sequence from the *Pichia pastoris* acid phosphatase gene. It also contains sequences from the *E. coli* vector pBR322 and sequences derived from the genome of *Pichia pastoris.* The essential features are the 5' region of the AOX gene including the methanol regulatable AOX promoter for high level transcription, the 3' region from the AOX gene containing the alcohol oxidase transcriptional terminator sequence, a further region from the 3' region which together with the 5'AOX region is required for site directed integration into the expression cassette into the chromosome. The *Pichia* histidinol dehydrogenase gene HIS4 is carried and used to complement the defective copy of the his4 gene on the host genome. The ampicillin resistance gene is carried to enable selection in the *E. coli* host used during the genetic manipulation.

The pHILS1 vector was manipulated to allow expression of the B2M gene under the control of the Pho1 secretion signal:

The gene encoding B2M was cloned into the pHILSI vector from the *S. cerevisiae* expression vector pSW6 containing the gene as a *Hind*III-*Bam*HI fragment (Example 2). The overhanging end of the DNA fragment at the *Hind*III site was first filled in to create a blunt end using the Klenow fragment of DNA polymerase I together with the required deoxynucleotide triphosphates according to standard methodology. The vector pHILSI was restricted with restriction endonucleases *EcoR1* and *Bam* H1. The overhanging end of the DNA fragment at the *Eco*R1 site was filled in as described above to create a blunt end. The B2M gene, as a blunt end *Bam*HI site was cloned into the blunt end *Bam*HI treated vector. The integrity of the resultant vector, intermediary vector 3, was confirmed by restriction analysis.

The *Hind*III-*Bam*HI fragment from the expression vector pSW6 contains the B2M gene fused at the 3' end to the last 5 amino acids of the yeast alpha factor which precedes the KEX2 cleavage site required for liberation of the mature peptide following secretion from the *Pichia pastoris* host when using the alpha factor sequence. This sequence and a few bases at the cloning site were deleted to allow the direct fusion of the Pho1 leader sequence and the B2M gene. The sequence deleted is (5'-CGAGAATTAGCTTGGATAAAAGA-3'), SEQ ID 24

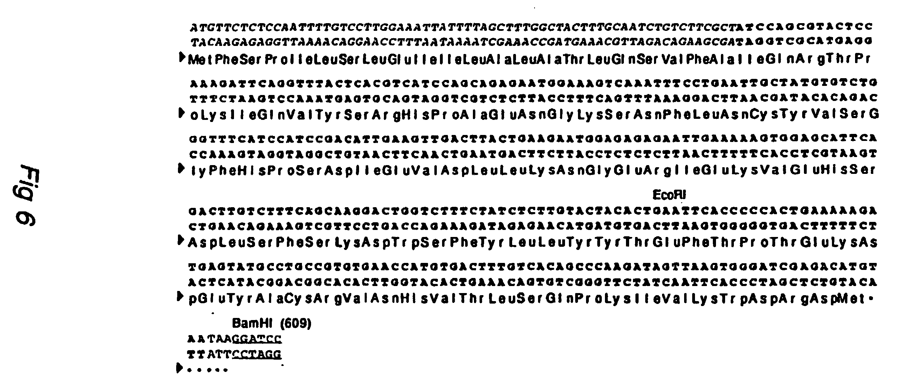

A ca 1150 bp (*Sst*I) *Sac*I*-Bam*HI fragment was isolated from the intermediary vector 3 and cloned into the vector M13mp18. This fragment contains 700bp of the 5' AOX promoter sequence, Pho1 signal sequence and the B2M gene. The M13 construct was then used for mutagenesis to delete the intervening sequence between the Pho1 sequence and the B2M gene. The oligonucleotide primer used for mutagenesis was (5'-GGAGTACGCTGGATAGCGAAGACAGATTG-3') SEQ ID 20. The mutants were identified by sequence analysis using the sequencing primer (5'-CCATTCTCTGCTGGATG-3') SEO ID 21. The sequence of Pho1/B2M is shown in Figure 6, SEQ ID 22

The vector was reconstructed using the expression vector pLH15 (Example 4(5)) as a backbone for the cloning. The vector was restricted with the restriction endonucleases (*Sst*) *Sac*l and *Bam*HI to release a ca 1150bp fragment containing a 700bp fragment of the 5' AOX promoter sequence and alpha factor signal sequence fused to the B2M gene, which was then replaced by the mutagenised (*Sst*) *Sac*I-*Bam*HI fragment containing a 700bp fragment of the 5' AOX promoter sequence, Pho1 signal sequence fused to the B2M gene. The resultant recombinant was called pLH46. The vector is shown on Figure 6. The integrity of the construct was confirmed by restriction analysis using the sequencing primer (5'-GCATTCTGACATCCTCT-3') SEQ ID 23

### Example 6 - Construction of Pichia expression strains

pLH15 and pLH46 plasmid DNA prepared as in Examples 5 and 6 were separately linearised by cutting with the restriction endonuclease Sac I. This was to enable the expression cassette to integrate via homologous recombination of sequences on the expression cassette and the host chromosome. In each case, the linearised plasmid was then transformed into *P.pastoris* strains GS115 (NRRL Y-1585) and KM71 which have the genotypes *his* 4 and *his4,* aox1::SARG4. The use of these strains is not critical for use either in this preparation or in the invention in general. Any suitable strain can be used, such as, for example, strain SMD1163 which has the genotype *his*4, *pr*B1, *pep*4. Strains GS115 and KM71 are described in Phillips patent number AU-B-63882/86 (Site selective genomic modification of yeast of the genus *Pichia).* Using the method described below the plasmid DNA was transformed into the host strain.

Briefly, yeast strain GS115 was grown overnight in 200mL of YEPD medium at 30°C on an orbital shaker. Cultures at an A₆₀₀ of between 0.1 and 0.3 were harvested by centrifugation at 3000rpm for 5min, washed in sterile water, recentrifuged, washed in SED buffer, recentrifuged, washed in 1M sorbitol, recentrifuged and resuspended in 20mL SCE buffer. Cells were then incubated at 30°C with the enzyme zymolyase to remove the cell wall. Spheroplasting was allowed to continue until approxiamately 70% of the cells had been turned into spheroplasts. These were then collected by gentle centrifugation (750xg 10min). Spheroplasts were then washed in 1M sorbitol and resuspended in 600uL CAS buffer. 100uL aliquots of spheroplasts were then incubated for 10mins with 10ug of the linearised DNA. 1mL of PEG buffer was then added and incubated for a further 10mins. After collecting the spheroplasts by gentle centrifugation and aspirating the PEG solution the cells were resuspended in 150uL of SOS medium and incubated for 20mins. After the addition of 850uL of 1M sorbitol the cells were ready for plating on regeneration agarose.

100uL of transformed spheroplasts were then added to 10mL of molten (42°C) agarose-sorbitol regeneration medium and poured onto agarose-sorbitol base plates and allowed to grow for 5-7 days at 30°C.

All yeast media and transformation buffers were as described in the appendix.

After 5-7 days transformants were collected along with the agarose overlay they had been growing in, transferred to a 50mL centrifuge tube and resusended in 50mM sodium phosphate buffer pH6 and after suitable mixing and agitation to remove the cells from the agarose they were diluted and plated onto YEPD agar plates containing the antibiotic G418 at concentrations between 0 and 2000 ug/mL. Only cells in which several copies of the expresssion cassette had integrated onto the host chromosome would be able to grow on high levels of antibiotic by virtue of the several copies of the kanamycin resistance gene they would be carrying. Such cells are deemed desirable since they will also be carrying several copies of the B2M gene. Previous work has shown such multicopy integrants to be high producers under conditions were the foreign gene is expressed (Clare,J.J., Rayment,F.B., Ballantine,S.P., Sreekrishna,K., and Romanos,M.A., BlO/TECHNOLOGY, 9, 455-460, 1991). Plates were incubated at 30°C for 5-7 days. Colonies occuring on plates containing high concentrations of the antibiotic were then picked and streaked onto fresh MD agar plates. Single colonies were obtained after 3-4 days growth at 30°c.

### Example 7 - Expression of B2M in Pichia pastoris

Single colonies of transformed strains were used to inoculate 5mL of BMGC medium and the cultures were grown overnight at 30°C on an orbital shaker. This 5mL overnight culture was then used to inoculate 2L baffled shake flasks containing 50mL of the medium BMGC. After 24h growth at 30°C on an orbital incubator cells were harvested by centrifugation at 3000rpm for 5min and resuspended in 50mL of BMMC. This induces gene expression from the AOX1 promoter. Induction was carried out by growth in the methanol containing medium at 30°C for 48 hours. After 24 hours 250µL of sterile methanol are added to the flask to replace that lost by evaporation.

After 48 hours the culture supernatant was collected by centrifugation at 3000rpm for 5min. The supernatant was used for further analysis. Production levels from strains with the alpha factor leader sequence were estimated by SDS-PAGE to be in the order of 150mg/L.

There were two bands visible on a Coomassie stained gel both of which react with the B2M specific monoclonal antibody BBM1 (ex Institute of Molecular Medicine, Oxford, England) on a Western blot. The larger band was apparently B2M with incomplete removal of the alpha factor leader. When used in a folding assay (Townsend *et al.* (1990) Cell. **62**, 285-295) the *Pichia* supernatant facilitates the folding of Class I molecules similar to commercially available material but at about half the level expected if all the *Pichia* expressed protein was native B2M.

N-terminal sequence of material from strains untilising the alpha factor leader sequence revealed that only about 30% of the material had the correct N-terminus. About 70% contained additional sequence derived from the leader sequence.

Production levels from strains utilising the Pho1 leader sequence were estimated to be about 100mg/L. There was only a single band visible on a Coomassie stained SDS-PAGE gel. N-terminal sequencing of this material revealed the >90% had the correct N-terminal sequence.

### Example 8 - Fermenter Expression of B2M in Pichia pastoris

An isolated colony of a strain shown to produce B2M from the Pho1 leader (Example 7) was inoculated into 5mL of YEPD medium and grown overnight at 30°C. This culture was used to inoculate 300mL of YEPGlycerol medium in a 2L baffled shake flask. This second culture was grown for 19h as above and then used to seed a 5L fermenter.

The fermentor was assembled with 3L of HCD(hh) medium, and autoclaved at 121°C for 45min. When cool, the fermentor was attached to its control and service modules and brought to its operating conditions of 30°C, pH 5.85 (controlled by the addition of '880' ammonia solution on demand), 800rpm agitation, 1vvm air flow. The medium was completed by the addition of biotin. The fermentor was inoculated with seed culture described above, and grown in a batch mode for 21 h. When the batch source (glycerol) was depleted (signalled by a sudden rise in DOT) a glycerol feed was started (500g/L glycerol, 5mL/L PTM1, 2mL/L 500xB) at 34mL/h (=17g glycerol/h). This feed ran for 6h and was then reduced to 24mUh (=12g glycerol/h) and a second methanol feed (methanol plus 5mL/L PTM1, 2mL/L 500xB) started at 6mL/h (=5g methanol/h). These two feeds ran for 18h at which point the fermentation was harvested.

Analysis of cell free supernatant by SDS-PAGE revealed the presence of a single band of the correct molecular weight. Comparison with a known standard revealed production levels to be of the order of 1g/L. N-terminal sequence of this material gave the correct sequence.

### Media recipies

| **BMGC** | |
|---|---|
| **Quantities per litre:** | |
| Sodium phosphate buffer 1M, | pH6 100mL |
| Casamino acids (100g/L) | 100mL |
| Yeast Nitrogen Base (13.4 g/L) | 100mL |
| Biotin (0.5g/L) | 2mL |
| Glycerol | 10mL |
| Filter sterilise | |

### BMMC

As above but replace glycerol with 5mL of methanol.

| YEPD | |
|---|---|
| Yeast extract | 10g/L |
| Peptone | 20g/L |
| Glucose | 10g/L |

### For solid medium add 15g/L agar

Autoclave at 121°C 15mins

### YEPGlycerol

As above but replace glucose with glycerol

| MD | |
|---|---|
| Yeast nitrogen base | 13.4g/L |
| Biotin | 0.4g/L |
| Glucose | 20g/L |
| Filter sterilise | |

### For solid medium add 15g/L agar

| HCD(hh) | |
|---|---|
| H3PO4 (85%) | 26mL/L |
| CaSO4.H2O | 1.1 g/L |
| K2SO4 | 17.9g/L |
| MgSO4.7H2O | 14.6g/L |
| KOH | 4.9g/L |
| Glycerol | 50g/L |

Add 4.5 mL of Strucktol JA673 anti-foam and 2.5ml PTM1 trace metals.

Sterilize in the fermentor and bring to pH 5.85 with '880' ammonia solution. Add 2.5m" od 500xB prior to inoculation.

| PTM1 trace element solution: | |
|---|---|
| CuSO4.5 H2O | 6g/L |
| Kl | 0.8gl |
| MnSO4.H2O | 3.0g/L |
| NaMoO4.2H2O | 0.2g/L |
| H3BO3 | 0.02g/L |
| CoCl2.6 H2O | 0.5g/L |
| ZnSO4 | 20g/L |
| H2SO4 | 5mL/L |
| FeSO4.7H2O | 65g/L |
| Biotin | 0.2g/L |

Filter sterilize.

### 500xB Biotin solution

0.2g/L biotin in water
Filter sterilize.

| **Transformation buffers and reagents** | |
|---|---|
| 1) | SED |
| Sorbitol | 1M |
| EDTA (pH8) | 25mM |
| DTT | 50mM (add just prior to use) |

| 2) SCE | |
|---|---|
| Sorbitol | 1M |
| EDTA | 1 mM |
| Sodium citrate buffer pH5.8 | 10mM |

| 3) CAS | |
|---|---|
| Sorbitol | 1M |
| Tris-Cl pH7.5 | 10mM |
| CaCl₂ | 10mM |

| 4) PEG solution) | |
|---|---|
| PEG 3350 | 200g/L |
| Tris-Cl pH7.5 | 10mM |
| CaCl₂ | 10mM |
| Prepare fresh and filter sterilise. Discard if pH is below 7. | |

| 5) SOS | |
|---|---|
| Sorbitol | 1M |
| YEPD | x0.3 |
| CaCl₂ | 10mM |

| 6) Regeneration medium (RD) | |
|---|---|
| Sorbitol | 186g/L |
| Agarose | 10g/L |
| Glucose | 20g/L |
| Yeast nitrogen base | 1.34g/L |
| Biotin | 400mg/L |
| Histidine assay medium* | 2g/L |
| Glutamic acid | 50mg/L |
| Methionine | 50mg/L |
| Lysine | 50mg/L |
| Leucine | 50mg/L |
| Isoleucine | 50mg/L |

| | |
|---|---|
| *DIFCO Ltd For base plates use agarose at 20g/L | |

## Claims

1. A method of producing recombinant beta-2-microglobulin (B2M) by cultivating a methylotropic yeast harbouring an expression vector comprising DNA encoding B2M, wherein the B2M is expressed as a fusion of B2M with a Pho1 secretary leader, and the resultant fusion is cleaved by endogenous endoproteinase(s) present in the growth medium to release B2M into the medium and recovering expressed B2M.

2. A method according to claim 1, wherein the yeast is *Pichia*

3. A method according to claim 2, wherein the *Pichia* is *Pichia pastoris.*

4. A method according to any one of the preceding claims, wherein the expression vector comprises a promoter selected from the promoters of AOX, PGK, GAPD, GAL1-10, PHO5, ADH1, CVG1 and the Tydelta sequences.

5. A method according to claim 4, wherein the promoter as the AOX promoter.

6. A method according to any one of the preceding claims wherein the expression vector is pLH46 as described in Example 5, and Fig. 5.

7. A method according to any one of the preceding claims wherein the B2M is expressed at a level of 1g/L.

## Patentansprüche

1. Verfahren zum Herstellen von rekombinantem Beta-2-Mikroglobulin (B2M) durch Züchten einer methylotrophen Hefe, die einen Expressionsvektor beherbergt, welcher für B2M codierende DNA umfasst, wobei das B2M als eine Fusion von B2M mit einer Pho1-sekretorischen Leader-Sequenz exprimiert wird und die resultierende Fusion durch im Wachstumsmedium vorhandene endogene Endoproteinase(n) gespalten wird, um B2M in das Medium freizusetzen, und Gewinnen des exprimierten B2M.

2. Verfahren nach Anspruch 1, wobei die Hefe *Pichia* ist.

3. Verfahren nach Anspruch 2, wobei die *Pichia Pichia pastoris* ist.

4. Verfahren nach einem der vorangehenden Ansprüche, wobei der Expressionsvektor einen Promotoren umfasst, gewählt aus den Promotoren AOX, PGK, GAPD, GAL1-10, PHO5, ADH1, CVG1 und den Ty-Delta-Sequenzen.

5. Verfahren nach Anspruch 4, wobei der Promotor der AOX-Promotor ist.

6. Verfahren nach einem der vorangehenden Ansprüche, wobei der Expressionsvektor pLH46 ist, wie in Beispiel 5 und Fig. 5 beschrieben.

7. Verfahren nach einem der vorangehenden Ansprüche, wobei das B2M in einer Menge von 1 g/l exprimiert wird.

## Revendications

1. Méthode de production de bêta-2-microglobuline (B2M) recombinante en cultivant une levure méthylotrophe contenant un vecteur d'expression comprenant un ADN codant pour B2M, dans laquelle la B2M est exprimée comme une fusion de B2M avec un leader de sécrétion Pho1, et la fusion résultante est clivée par une (des) endoprotéinase(s) endogène(s) présente(s) dans le milieu de culture pour libérer B2M dans le milieu et en récupérant B2M exprimée.

2. Méthode selon la revendication 1, dans laquelle la levure est *Pichia.*

3. Méthode selon la revendication 2, dans laquelle le *Pichia* est *Pichia pastoris.*

4. Méthode selon l'une quelconque des revendications précédentes, dans laquelle le vecteur d'expression comprend un promoteur choisi parmi les promoteurs AOX, PGK, GAPD, GAL1-10, PHO5, ADH1, CVG1 et les séquences Tydelta.

5. Méthode selon la revendication 4, dans laquelle le promoteur est le promoteur AOX.

6. Méthode selon l'une quelconque des revendications précédentes, dans laquelle le vecteur d'expression est pLH46 comme décrit dans l'exemple 5, et la figure 5.

7. Méthode selon l'une quelconque des revendications précédentes dans laquelle la B2M est exprimée au taux de 1g/L.
